## Europäisches Patentamt
## European Patent Office
## Office européen des brevets

(19)

(11) Publication number: **0 002 645**
**B1**

(12) **EUROPEAN PATENT SPECIFICATION**

(45) Date of publication of patent specification: **06.01.82**

(51) Int. Cl.³: **A 61 K 39/02**

(21) Application number: **78400245.3**

(22) Date of filing: **15.12.78**

(54) Antigenic complex from neisseria gonorrhoeae, process and vaccine.

(30) Priority: **20.12.77 US 862265**

(43) Date of publication of application:
**27.06.79 Bulletin 79/13**

(45) Publication of the grant of the European patent:
**06.01.82 Bulletin 82/1**

(84) Designated Contracting States:
**BE CH DE FR GB NL**

(56) References cited:
CHEMICAL ABSTRACTS, vol. 84, no. 25, June 21, 1976, ref. 178110h
Columbus, Ohio, USA
K. H. JOHNSTON et al., "The serological classification of Neisseria gonorrhoeae. I. Isolation of the outer membrane complex responsible for serotypic specificity", page 411.

CHEMICAL ABSTRACTS, vol. 87, no. 13, September 26, 1977, ref. 100438p
Columbus, Ohio, USA

(73) Proprietor: **MERCK & CO. INC.**
**126, East Lincoln Avenue P.O. Box 2000**
**Rahway, New Jersey 07065 (US)**

(72) Inventor: **Karkhanis, Yashwant Dattatraya**
**160 Coriell Avenue**
**Fanwood New Jersey 07023 (US)**
Inventor: **Carlo, Dennis John**
**195 West Lincoln Avenue**
**Rahway New Jersey 07065 (US)**

(74) Representative: **Corre, Jacques Denis Paul et al,**
**Cabinet Regimbeau 26, Avenue Kléber**
**F-75116 Paris (FR)**

(56) References cited:
T. M. BUCHANAN et al., "Immunity to gonococcal infection induced by vaccination with isolated outer membranes of Neisseria gonorrhoeae in guinea pigs", page 470.

CHEMICAL ABSTRACTS, vol. 87, no. 15, October 10, 1977, ref. 114215a
Columbus, Ohio, USA
J. E. HECKELS, "The surface properties of Neisseria gonorrhoeae: isolation of the major components of the outer membrane", page 303.

## Antigenic complex from neisseria gonorrhoeae, process and vaccine

It is known from article of K. H. Johnston et al., J. Exp. Med., 1976 143(4), 741—58, that some major outer membrane protein antigen may be extracted from *Neisseria gonorrhoeae* in order to use such specific antigen as the basis of a serotyping system for the gonococcus.

But because of the specificity such antigen cannot be used as a basis for vaccine effective against a broad spectrum of *N. gonorrhoeae* of various serotypes.

This is the purpose of invention to provide a complex which may be effective against *N. gonorrhoeae* of various serotypes.

Invention concerns the antigenic, immunogenic complex obtained from the cell surface of *N. gonorrhoeae* which is protective against *N. gonorrhoeae*, having a molecular weight of from about 9,200,000 to about 9,500,000, over 80% of the complex being formed of five subunits in approximately equal amount having molecular weight of about 68,000, about 50,000, about 34,000, about 27,000 and about 10,500, the complex containing on a dry weight basis from about 90 to about 95% protein, up to about 7% lipopolysaccharide, about 2% carbohydrate and less than 1% of RNA, DNA and phospholipids.

The invention deals also with a process for preparation of such a complex which comprises stirring a homogenized cell paste of *N. gonorrhoeae* for from about 10 to 15 hours, removing cellular debris, RNA and DNA from the homogenized extract, and separating a fraction having a molecular weight in excess of about 9,000,000.

The Melvin strain, Type 1, of *N. gonorrhoeae* used in this study was obtained from Mr. W. J. Brown at the Communicable Disease Center, Atlanta, Georgia. An unrestricted deposit of this organism was made with the American Type Culture Collection on 13 December 1977 under Accession N° 31356. The bacteria are grown in agar and cells from the agar are used to inoculate a fermentor. The cells are harvested at the stationary phase of growth and stored at −20°C if not used immediately.

The cell paste is homogenized in a salt solution at a pH of from about 6.8 to about 8.3 for a time insufficient to cause lysis. The resulting mixture is stirred, preferably at lowered temperature, typically from about 4 to about 8°C for several hours, typically for from about 10 to 15 hours. Any salt solution may be used as long as it is non-injurious to the cell surface of *N. gonorrhoeae*. Examples of suitable salts are Tris-HCl, phosphate buffered saline, LiCl, and physiological saline, or LiCl. Tris-HCl is preferred.

The stirred mixture is then centrifuged to remove cellular debris. Typically the pellet is removed at from about 30,000 to about 50,000 × g for from about 15 minutes to about 1 hour. In general longer times are employed with lower g and shorter times are employed with higher g. The supernatant resulting from the centrifugation is then treated to remove RNA and DNA. This may be effected by enzymatic digestion e.g., RNase and DNase. The digested mixture is subjected to treatment effective to fractionate molecules based on their molecular weight. As examples of such treatments there may be mentioned gel filtration, ultrafiltration, high speed ultracentrifugation or density gradient separation. Gel filtration is a particularly suitable method for this fractionation. The antigenically active fraction from the molecular weight fractionation is the desired immunogenic non-toxic complex of the present invention.

The antigenic complex has a molecular weight of from about 9,200,000 to about 9,500,000 consisting essentially of several subunits ranging in molecular weight from about 100,000 to about 10,500. There are 5 major bands present in approximately equal amounts of molecular weight about 68,000, about 50,000, about 34,000, about 27,000 and about 10,500. These five bands account for over 80% of the complex. On a dry weight basis the antigen contains from about 90 to about 95% protein (Lowry method using bovine serum albumin as a standard), not more than about 10%, lipopolysaccharide, and typically not more than about 5% (Perry et al., Can J. Biochem., *53*, 623 (1975), about 2% carbohydrate and less than 1% of RNA, DNA and phospholipids. RNA and DNA are assayed by the procedure of Schneider, *Methods Enzymol, 3*, 680 (1957), and phospholipids by the procedure of *Ames Methods Enzymol., 8*, 115 (1966).

The antigenic, immunogenic complex from the cell surface of *N. gonorhoeae* may be sterilized by filtration or treatment by chemicals such as thimerosol, phenol, formaldehyde and the like, and subdivided into a suitable container by distribution and administration as a vaccine. It may be administered in a suitable physiologically acceptable medium such as, for example, water for injection, saline, phosphate buffered saline, and the like. It may be combined with adjuvants such as, for example, as disclosed in U.S. patent 3,983,228 issued 28 September 1976 and carriers, e.g., alum.

The following examples illustrate the present invention without, however, limiting the same thereto.

### Example 1

A culture of *N. gonorrhoeae*, Melvin strain, ATCC 31356, is used to inoculate agar culture overnight. Cells growing on the agar culture are used to inoculate a 2 liter flask which is grown overnight and the contents used to inoculate a 14 liter fermentor which is grown overnight and the contents used to inoculate a 200 liter fermentor containing Frantz medium [J. Bact., *43*, 757, (1942)]. The fermentation is conducted at a temperature of 37°C and is stopped with the bacteria reach their stationary phase of growth. To the fermentation is added thimerosol at a concentration of 1:10,000. The cells are centrifuged in a Sharpless centrifuge and the cell paste is collected.

Approximately 30 g of cell paste are homogenized in 100 ml of Tris-HCl buffer in a Sorvall Omni-mixer for 1—2 minutes at a speed setting of 2. The mixture is transferred to a flask and the homogenization flask is washed with an additional 100 ml of Tris-HCl buffer. The mixture is stirred at 4°C for 15 hours. After extraction, the material is centrifuged at 39,100 ×g for 30 minutes in a Sorvall RC-2 centrifuge, and the residue is discarded. The supernatant is made 20 mM in $MgCl_2$ and treated with RNase and DNase (10 μg/ml) at 37°C for 45 minutes. The digested material is applied to an Agarose A-1.5 chromatography column and the fractions recovered from the column are tested for presence of antigenic material by the Ouchterlony immunodiffusion assay technique using antibody obtained as described in Example 2. Fractions containing antigenic material are pooled and applied to a Sepharose 6B chromatography column. The column is eluted with 50 mM ammonium bicarbonate buffer, pH 8.5 and the fractions tested for presence of antigenic material as indicated above. The active fraction yields 100 mg of an antigenic, immunogenic non-toxic complex having a purity of from 90—95%.

## Example 2

Live cells of *N. gonorrhoeae*, Melvin strain, ATCC 31356 are inoculated intravenously into a group of 4 New Zealand white rabbits. The procedure consists of intravenous inoculation on days 1, 3, 10 and 14 and bleeding 7 days later. This is followed by a rest period of one month after which the animals receive 2 additional injections on days 3 and 7 with bleeding 9 days later. Sera with good antibody activity are obtained.

Antisera against the purified antigen preparation of Example 1 is prepared by intramuscular inoculation in a group of 3 rabbits. Initial inoculation consists of 500—100 μg of antigen emulsified in an equal volume of complete Freund's adjuvant. Subsequent booster doses of equal amounts of antigen are emulsified in an equal volume of incomplete Freund's adjuvant and administered intramuscularly. The booster doses are given every three weeks. Rabbits are routinely bled on the 7th and 14th day and receive a booster of antigen following the bleeding. Sera with good antibody activity are obtained following the second injection.

## Example 3

The antigenically active material of Example 1, 250 μg, is injected into a group of 10 CD-1 albino male mice weighing approximately 10 g. A second group of 10 mice serve as control. After one week the animals in each group are challenged with $10^7$ live cells of *N. gonorrhoeae*. Fifty replicates of the foregoing procedure are carried out. Ninety to one hundred percent of the animals in the control groups are found to die within 24 hours whereas 90—100% of the animals in the group receiving the antigenic material prior to challenge survive.

## Example 4

Saline, 20 ml is added to 50 mg of the complex obtained in Example 1 and the resulting suspension is sterile filtered through a 0.45 μ micropore filter and asceptically filled into forty 0.1 ml vials. Each vial contains 0.25 mg of complex. The vials are stored at −20°C until used.

## Example 5

A culture (10 grams, wet weight) of *N. gonorrhoeae* cell is suspended in 200 ml of 0.2M LiCl. The cells are homogenized for about 1 minute at setting 2 of a Sorvall Omnimixer. The cells are extracted at 45°C for 2 hours at 200 rpm in a gyratory shaker. The extract is centrifuged at 10,000 rpm (9,000 ×g) and the supernatant aspirated. To the recovered supernatant, 580 ml, there is added 117.9 mg of $MgCl_2$ and 5.8 mg of DNase and 0.42 ml of RNase. The resulting mixture is stirred for 30 minutes at 37°C in a gyratory water bath shaker at setting 3. The mixture is then dialyzed overnight with 2 changes of water. The dialyzed liquid is lyophilized to yield 769.72 mg of crude gonococcal extract. The lyophilized extract is dissolved in 770 ml of distilled water, stirred and centrifuged at 13,000 rpm for 30 minutes. The supernatant is then applied in an Agarose A-5m column and the column eluted with 0.15 M NaCl. The material eluted from the column having an adsorption at 230 nm is pooled, dialyzed and lyophilized to yield the purified gonococcal complex.

Four groups of mice are vaccinated subcutaneously with 250 μg of the purified gonococcal antigen. A fifth group of unvaccinated mice serves as control. Eight days after vaccination, the mice are challenged with $1 \times 10^7$ live virulent *N. gonorrhoeae* cells. The percent of protection is determined by observation of the number of survivors after 48 hours. The following results are obtained:

| Group | No. in Group | No. Dead | % Protection |
|---|---|---|---|
| 1 | 10 | 0 | 100 |
| 2 | 10 | 0 | 100 |
| 3 | 7 | 1 | 90 |
| 4 | 10 | 2 | 80 |
| 5 (control) | 10 | 8 | 20 |

## Claims

1. An antigenic, immunogenic, complex obtained from the cell surface of *N. gonorrhoeae* which is protective against *N. gonorrhoeae*, having a molecular weight of from about 9,200,000 to about 9,500,000, over 80% of the complex being formed of five subunits in approximately equal amount having molecular weight of about 68,000, about 50,000, about 34,000, about 27,000 and about 10,500, the complex containing on a dry weight basis from about 90 to about 95% protein, up to about 7% lipopolysaccharide, about 2% carbohydrate and less than 1% of RNA, DNA and phospholipids.

2. An antigenic, immunogenic, complex according to claim 1 obtained from the cell surface of *N. gonorrhoeae* Melvin strain type 1 ATCC 31 356.

3. A composition comprising the complex of claim 1 or 2 in combination with a physiologically acceptable medium.

4. A composition according to claim 3 wherein the physiologically acceptable medium is saline.

5. A vaccine comprising the complex of claim 1 or 2 in sterile form in combination with a physiologically acceptable medium.

6. A vaccine according to claim 5 wherein the physiologically acceptable medium is saline.

7. A method of obtaining an antigenic, immunogenic complex according to claim 1 which comprises stirring a homogenized cell paste of *N. gonorrhoeae* for from about 10 to about 15 hours, removing cellular debris, RNA and DNA from the homogenized extract, and separating a fraction having a molecular weight in excess of about 9,000,000.

8. A method according to claim 7 wherein the *N. gonorrhoeae* is the Melvin strain ATCC 31 356.

9. A method according to claim 7 or 8 wherein the cell paste is homogenized at a pH from about 6,8 to about 8,3.

10. A method according to claim 7 or 8 wherein the cellular debris is removed by centrifugation.

11. A method according to claim 10 wherein the centrifugation is carried out at from about 3,000 ×g to about 50,000 ×g.

12. A method according to claim 7 or 8 wherein the RNA and DNA are removed by treatment with an enzyme.

13. A method according to claim 7 or 8 wherein the fraction is separated by gel filtration.

## Revendications

1. Complexe immunogène antigénique provenant de la surface cellulaire de *N. gonorrhoeae* protégeant contre *N. gonorrhoeae*, ayant un poids moléculaire d'environ 9 200 000 à environ 9 500 000, plus de 80% du complexe étant formé de 5 sous-unités en quantités approximativement égales ayant un poids moléculaire d'environ 68 000, environ 50 000, environ 34 000, environ 27 000 et environ 10 500, le complexe contenant, sur la base du poids sec, d'environ 90 à environ 95% de protéine, jusqu'à environ 7% de lipopolysaccharide, environ 2% d'hydrate de carbone et moins de 1% d'ARN, d'ADN at de phospholipides.

2. Complexe immunogène antigénique selon la revendication 1 obtenu à partir de la surface cellulaire de *N. gonorrhoeae* souche Melvin type 1 ATCC 31 356.

3. Composition contenant le complexe de la revendication 1 ou de la revendication 2 combiné avec un milieu physiologiquement acceptable.

4. Composition selon la revendication 3 où le milieu physiologiquement acceptable est le sel physiologique.

5. Vaccin comprenant le complexe de la revendication 1 ou 2 sous forme stérile combiné avec un milieu physiologiquement acceptable.

6. Vaccin selon la revendication 5 où le milieu physiologiquement acceptable est le sel physiologique.

7. Procédé d'obtention d'un complexe immunogène antigénique selon la revendication 1 qui consiste à agiter une pâte cellulaire homogénéisée de *N. gonorrhoeae* pendant environ 10 à environ 15 heures, à retirer les débris cellulaires, l'ARN et l'ADN de l'extrait homogénéisé, et à séparer une fraction ayant un poids moléculaire dépassant environ 9 000 000.

8. Procédé selon la revendication 7 où le *N. gonorrhoeae* est le souche Melvin ATCC 31 356.

9. Procédé selon les revendications 7 ou 8 où l'on homogénéise la pâte cellulaire à un Ph d'environ 6,8 à environ 8,3.

10. Procédé selon les revendications 7 ou 8 où l'on retire les débris cellulaires par centrifugation.

11. Procédé selon la revendication 10 où la centrifugation s'effectue entre environ 30 000 g et environ 50 000 g.

12. Procédé selon les revendications 7 ou 8 où l'on retire l'ARN et l'ADN par traitement avec une enzyme.

13. Procédé selon les revendications 7 ou 8, où l'on sépare la fraction par filtration sur gel.

## Patentansprüche

1. Antigenischer, immunogener Komplex, erhalten aus der Zelloberfläche von N. gonorrhoeae, der schützend wirkt gegen N. gonorrhoeae, mit einem Molekulargewicht von etwa 9 200 000 bis etwa 9 500 000, wobei über 80% des Komplexes aus 5 Untereinheiten in ungefähr gleicher Menge mit einem Molekulargewicht von etwa 68 000, etwa 50 000, etwa 34 000, etwa 27 000 und etwa 10 500 gebildet sind, der Komplex auf Trockengewichtsbasis etwa 90 bis etwa 95% Protein, bis zu etwa 7% Lipopolysaccharid, etwa 2% Kohlenhydrat und weniger als 1% RNA, DNA und Phospholipide enthält.

2. Antigenischer, immunogener Komplex nach Anspruch 1, erhalten aus der Zelloberfläche von N. gonorrhoeae Melvin-Stamm Typ 1 ATCC 31 356.

3. Zusammensetzung, enthaltend den Komplex nach Anspruch 1 oder 2 in Kombination mit einem physiologisch annehmbaren Medium.

4. Zusammensetzung nach Anspruch 3, in der das physiologisch annehmbare Medium Salzlösung ist.

5. Impfstoff, enthaltend den Komplex nach Anspruch 1 oder 2 in steriler Form in Kombination mit einem physiologisch annehmbaren Medium.

6. Impfstoff nach Anspruch 5, in der das physiologisch annehmbare Medium Salzlösung ist.

7. Ein Verfahren zur Herstellung eines antigenen, immunogenen Komplexes nach Anspruch 1, bei dem eine homogenisierte Zellpaste von N. gonorrhoeae während etwa 10 bis etwa 15 Stunden gerührt wird, Zellbruchstücke, RNA und DNA aus dem homogenisierten Extrakt entfernt werden und eine Fraktion mit einem Molekulargewicht von über etwa 9 000 000 abgetrennt wird.

8. Ein Verfahren nach Anspruch 7, bei dem N. gonorrhoeae der Melvin-Stamm ATCC 31 356 ist.

9. Ein Verfahren nach Anspruch 7 oder 8, bei dem die Zellpaste bei einem pH-Wert von etwa 6,8 bis etwa 8,3 homogenisiert wird.

10. Ein Verfahren nach Anspruch 7 oder 8, bei dem die Zellbruchstücke durch Zentrifugieren entfernt werden.

11. Ein Verfahren nach Anspruch 10, bei dem das Zentrifugieren bei etwa 30 000 ×g bis etwa 50 000 ×g durchgeführt wird.

12. Ein Verfahren nach Anspruch 7 oder 8, bei dem RNA und DNA durch Behandeln mit einem Enzym entfernt werden.

13. Ein Verfahren nach Anspruch 7 oder 8, bei dem die Fraktion durch Gelfiltration abgetrennt wird.